(19) Europäisches Patentamt
European Patent Office
Office européen des brevets

(11) Veröffentlichungsnummer: **0 209 779**
**A2**

# (12) EUROPÄISCHE PATENTANMELDUNG

(21) Anmeldenummer: 86109232.8

(22) Anmeldetag: 07.07.86

(51) Int. Cl.[4]: C 07 D 239/46
C 07 D 239/42, C 07 D 239/52

(30) Priorität: 20.07.85 DE 3525977

(43) Veröffentlichungstag der Anmeldung: 28.01.87 Patentblatt 87/5

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Kluth, Joachim, Dr.
Kurt-Schumacher-Strasse 9
D-4018 Langenfeld(DE)

(72) Erfinder: Müller, Klaus-Helmut, Dr.
Bockhackstrasse 55
D-4000 Düsseldorf 13(DE)

(72) Erfinder: Pfister, Theodor, Dr.
Lichtenberger Strasse 30
D-4019 Monheim(DE)

## (54) Verfahren zur Herstellung von 2-Cyanamino-pyrimidin-Derivaten.

(57) Die Erfindung betrifft ein neues Verfahren zur Herstellung von 2-Cyanamino-pyrimidin-Derivaten der allgemeinen Formel (I)

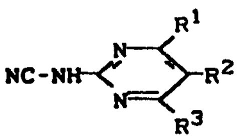

(I)

in welcher

$R^1$ für Wasserstoff, Halogen, gegebenenfalls substituiertes Alkyl oder gegebenenfalls substituiertes Alkoxy steht,

$R^2$ für Wasserstoff, Alkyl oder Halogen steht und

$R^3$ für Wasserstoff, Halogen, gegebenenfalls substituiertes Alkyl oder gegebenenfalls substituiertes Alkoxy steht,

das dadurch gekennzeichnet ist, daß man 2-Alkylsulfonyl-pyrimidin-Derivate der allgemeinen Formel (II)

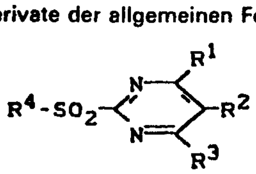

(II)

in welcher

$R^1$, $R^2$ und $R^3$ die oben angegebenen Bedeutungen haben und

$R^4$ für gegebenenfalls substituiertes Alkyl steht,

mit Cyanamid oder mit Metallsalzen von Cyanamid, gegebenenfalls in Gegenwart eines Katalysators, gegebenenfalls in Gegenwart eines Säureakzeptors und gegebenenfalls in Gegenwart eines Verdünnungsmittels, bei Temperaturen zwischen 0 und 150 °C umsetzt.

Die 2-Cyanamino-pyrimidin-Derivate (I) können als Zwischenprodukte zur Herstellung von bekannten Herbiziden und Pflanzenwachstumsregulatoren verwendet werden.

Croydon Printing Company Ltd.

BAYER AKTIENGESELLSCHAFT 5090 Leverkusen, Bayerwerk
Konzernverwaltung RP
Patentabteilung Bi/cm/c
IVa/ZP

# Verfahren zur Herstellung von 2-Cyanamino-pyrimidin-Derivaten

Die Erfindung betrifft ein neues Verfahren zur Herstellung von 2-Cyanamino-pyrimidin-Derivaten, welche als Zwischenprodukte für die Herstellung von Herbiziden und Pflanzenwachstumsregulatoren verwendet werden können.

Es ist bereits bekannt, daß man Cyanamino-heteroarene erhält, wenn man entsprechende Halogen-heteroarene mit Cyanamid-Salzen umsetzt (vergl. DE-OS 3 334 455; EP-A-0 121 082). Bei diesen Umsetzungen sind jedoch Qualität und Ausbeute der Produkte in vielen Fällen unbefriedigend.

Es ist weiter bekannt, daß man bestimmte Cyanamino-pyrimidine erhält, wenn man β-Dicarbonylverbindungen mit Cyanoguanidin ("Dicyandiamid") umsetzt (vergl. DE-OS 3 334 455). Hierbei erhält man jedoch 2-Cyanamino-4-hydroxy-pyrimidin-Derivate, deren Umwandlung in die als Herbizid-Zwischenprodukte interessanten 2-Cyanamino-4-alkoxy-pyrimidin-Derivate sehr schwierig und oft mit unbefriedigendem Ergebnis durchzuführen ist.

Es wurde nun gefunden, daß man 2-Cyanamino-pyrimidin-Derivate der allgemeinen Formel (I)

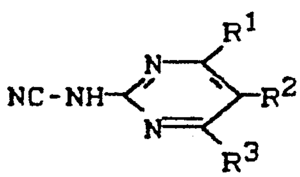

(I)

in welcher

$R^1$ für Wasserstoff, Halogen, gegebenenfalls substituiertes Alkyl oder gegebenenfalls substituiertes Alkoxy steht,

$R^2$ für Wasserstoff, Alkyl oder Halogen steht und

$R^3$ für Wasserstoff, Halogen, gegebenenfalls substituiertes Alkyl oder gegebenenfalls substituiertes Alkoxy steht,

erhält, wenn man 2-Alkylsulfonyl-pyrimidin-Derivate der allgemeinen Formel (II)

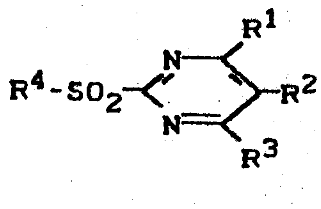

(II)

in welcher

$R^1$, $R^2$ und $R^3$ die oben angegebenen Bedeutungen haben und

$R^4$ für gegebenenfalls substituiertes Alkyl steht,

mit Cyanamid oder mit Metallsalzen von Cyanamid,

gegebenenfalls in Gegenwart eines Katalysators, gegebenenfalls in Gegenwart eines Säureakzeptors und gegebenenfalls in Gegenwart eines Verdünnungsmittels, bei Temperaturen zwischen 0 und 150 °C umsetzt.

Überraschenderweise können nach dem erfindungsgemäßen Verfahren 2-Cyanamino-pyrimidin-Derivate auf einfache Weise in höheren Ausbeuten und besserer Qualität als nach bekannten Methoden hergestellt werden.

Bevorzugt werden mit Hilfe des erfindungsgemäßen Verfahrens die Verbindungen der Formel (I) hergestellt, in welcher

$R^1$ für Wasserstoff, Fluor, Chlor, Brom, $C_1$-$C_4$-Alkyl [welches gegebenenfalls durch Fluor, Chlor, Brom oder $C_1$-$C_2$-Alkoxy substituiert ist] oder $C_1$-$C_4$-Alkoxy [welches gegebenenfalls durch Fluor, Chlor, Brom oder $C_1$-$C_2$-Alkoxy substituiert ist] steht,

$R^2$ für Wasserstoff, Methyl, Fluor, Chlor oder Brom steht und

$R^3$ für Wasserstoff, Fluor, Chlor, Brom, $C_1$-$C_4$-Alkyl oder $C_1$-$C_4$-Alkoxy [welche gegebenenfalls durch Fluor, Chlor, Brom oder $C_1$-$C_2$-Alkoxy substituiert sind] steht.

Besonders bevorzugt werden diejenigen Verbindungen der Formel (I) hergestellt, in welcher

$R^1$ für Wasserstoff, Methyl, Ethyl, Chlor, Trifluormethyl, Methoxy, Ethoxy oder Difluormethoxy steht,

$R^2$ für Wasserstoff oder Chlor steht und

$R^3$ für Wasserstoff, Chlor, Methyl, Ethyl, Trifluormethyl, Methoxy, Ethoxy oder Difluormethoxy steht.

Verwendet man als Ausgangsstoffe beispielsweise 5-Chlor-4-methoxy-6-methyl-2-methylsulfonyl-pyrimidin und Natriumcyanamid, so kann der Reaktionsablauf beim erfindungsgemäßen Verfahren durch das folgende Formelschema wiedergegeben werden:

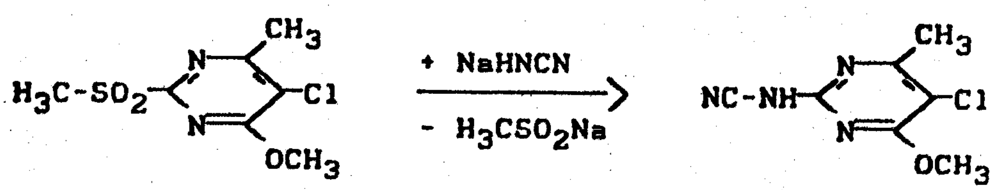


Die als Ausgangsstoffe zu verwendenden 2-Alkylsulfonyl-pyrimidin-Derivate sind durch die Formel (II) allgemein definiert. Vorzugsweise stehen darin

$R^1$ für Wasserstoff, Fluor, Chlor, Brom, $C_1$-$C_4$-Alkyl [welches gegebenenfalls durch Fluor, Chlor, Brom oder $C_1$-$C_2$-Alkoxy substituiert ist] oder $C_1$-$C_4$-Alkoxy [welches gegebenenfalls durch Fluor, Chlor, Brom oder $C_1$-$C_2$-Alkoxy substituiert ist],

$R^2$ für Wasserstoff, Methyl, Fluor, Chlor oder Brom,

$R^3$ für Wasserstoff, Fluor, Chlor, Brom, $C_1$-$C_4$-Alkyl oder $C_1$-$C_4$-Alkoxy [welche gegebenenfalls durch

Fluor, Chlor, Brom oder $C_1$-$C_2$-Alkoxy substituiert sind] und

$R^4$ für $C_1$-$C_4$-Alkyl oder Benzyl.

Insbesondere bevorzugt sind Ausgangsstoffe der Formel (II), in welcher

$R^1$ für Wasserstoff, Methyl, Ethyl, Chlor, Trifluormethyl, Methoxy, Ethoxy oder Difluormethoxy steht,

$R^2$ für Wasserstoff oder Chlor steht,

$R^3$ für Wasserstoff, Chlor, Methyl, Ethyl, Trifluormethyl, Methoxy, Ethoxy oder Difluormethoxy steht und

$R^4$ für Methyl steht.

Als Beispiele für die Verbindungen der Formel (II) seien genannt:

2-Methylsulfonyl-pyrimidin, 2-Ethylsulfonyl-pyrimidin,

2-Benzylsulfonyl-pyrimidin, 4,6-Dimethoxy-, 4,6-Dimethyl-, 4-Methyl-, 4-Methoxy-6-methyl-, 4-Ethoxy-6-methyl-, 4-Chlor-6-methyl-, 4-Chlor-6-methoxy-, 4-Difluormethoxy-6-methyl-, 4-Methyl-6-methylthio-, 4-Chlor-6-ethoxy-, 4-Chlor-6-methylthio-, 4-Ethyl-, 4,6-Diethyl-, 4,6-Diethoxy-, 5-Chlor-6-methyl-, 5-Chlor-4-methoxy-6-methyl-, 5-Chlor-4-ethoxy-6-methyl-, 4,5-Dichlor-6-methyl-, 4,5-Dichlor-6-methoxy-, 4-Methoxy-, 4-Ethoxy- und 4,6-Dichlor-2-methylsulfonyl-pyrimidin, -2-ethylsulfonyl-pyrimidin und -2-benzylsulfonyl-pyrimidin.

Die als Ausgangsstoffe zu verwendenden 2-Alkylsulfonyl-pyrimidin-Derivate der Formel (II) sind bekannt und/ oder können nach an sich bekannten Verfahren hergestellt werden (vergl. J. Chem. Soc. 1957, 1830 - 1833; DE-OS 2 501 769; US-PS 3 308 119; J. Org. Chem. 26 (1961), 792).

Man erhält die Verbindungen der Formel (II), wenn man 2-Alkylthio-pyrimidin-Derivate der Formel (III)

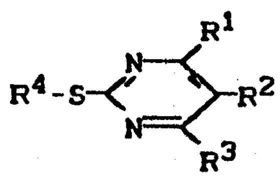 (III)

in welcher

$R^1$, $R^2$, $R^3$ und $R^4$ die oben angegebenen Bedeutungen haben,

mit Oxidationsmitteln, wie z. B. Chlorwasser bzw. hypochloriger Säure oder deren Metallsalzlösungen in Wasser, gegebenenfalls in Gegenwart von organischen Lösungsmitteln wie z. B. Methylenchlorid, Chloroform, Tetrachlormethan oder Chlorbenzol, bei Temperaturen zwischen -20 °C und +30 °C, vorzugsweise zwischen -5 °C und +10 °C umsetzt.

Die Aufarbeitung kann nach üblichen Methoden durchgeführt werden. Soweit die Produkte der Formel (II) kristallin anfallen, können sie durch Absaugen isoliert werden. Die Verbindungen der Formel (II) können aber auch in organischen Lösungsmitteln wie z. B. Methylenchlorid, Chloroform oder Tetrachlormethan aufgenommen werden. Man erhält dann diese Verbindungen nach Waschen der organischen Lösung mit Wasser, Trocknen, Filtrieren und Einengen in genügender Qualität für weitere Umsetzungen.

Die als Zwischenprodukte benötigten 2-Alkylthio-pyrimidin-Derivate der Formel (III) sind bekannt und/oder können nach an sich bekannten Verfahren hergestellt werden (vergl. J. Chem. Soc. 1957, 1830 - 1833; Chem. Pharm. Bull. 5 (1958), 479 - 482, ibid. 11 (1963), 1382 - 1388; US-PS 4 199 583; US-PS 3 308 119; J. Org. Chem. 26 (1961), 792).

Das erfindungsgemäße Verfahren wird vorzugsweise in Gegenwart eines Verdünnungsmittels durchgeführt. Als solche kommen praktisch alle inerten organischen Lösungsmittel, insbesondere jedoch aprotisch polare Solventien in Betracht. Hierzu gehören chlorierte Kohlenwasserstoffe wie z. B. Methylenchlorid, Chloroform oder Chlorbenzol, Ketone wie z. B. Aceton, Methylethylketon, Methylisopropylketon und Methylisobutylketon, Nitrile wie z. B. Acetonitril und Propionitril, Ether wie z. B. Diethylether, Diisopropylether, Dimethoxyethan, Tetrahydrofuran und Dioxan, Amide wie z. B. Dimethylformamid und Dimethylacetamid sowie Dimethylsulfoxid und Sulfolan.

Aus der genannten Gruppe von aprotisch polaren Lösungsmitteln sind die folgenden besonders bevorzugt: Dimethylformamid, Dimethylacetamid, Acetonitril und Propionitril.

Das erfindungsgemäße Verfahren wird gegebenenfalls in Gegenwart eines Katalysators durchgeführt. Als Katalysatoren kommen hierbei vorzugsweise Verbindungen in Betracht, welche zum Phasentransfer von Anionen geeignet sind. Als Beispiele seien genannt:

Benzyltriethylammoniumchlorid (TEBA), Tetrabutylammoniumbromid, Benzyldimethylhexadecylammoniumchlorid, Benzyldimethyltetradecylammoniumchlorid, Benzyltributylammoniumchlorid und Dodecylethyldimethylammoniumbromid.

Das erfindungsgemäße Verfahren wird gegebenenfalls in Gegenwart eines Säureakzeptors durchgeführt. Es kommen vorzugsweise Verbindungen in Betracht, welche eine vergleichsweise geringe Tendenz zu nucleophilen Reaktionen zeigen. Hierzu gehören Alkalimetall- und Erdalkalimetall-hydride wie z. B. Natrium-, Kalium-, und Calciumhydrid, Alkalimetall- und Erdalkalimetall-carbonate wie z. B. Natriumcarbonat, Kaliumcarbonat und Calciumcarbonat sowie aprotische Amine wie z. B. Pyridin, Tributylamin, N,N-Dimethylbenzylamin und Diazabicycloundecen.

Für das erfindungsgemäße Verfahren kann Cyanamid in verschiedenen Qualitäten eingesetzt werden. Es ist auch die Verwendung von Metallsalzen von Cyanamid wie z. B. Natriumcyanamid, Dinatriumcyanamid, Kaliumcyanamid, Dikaliumcyanamid und Calciumcyanamid möglich.

Die Reaktionstemperaturen können beim erfindungsgemäßen Verfahren innerhalb eines größeren Bereichs varriiert werden. Im allgemeinen arbeitet man zwischen 0 °C und 150 °C, vorzugsweise zwischen 10 °C und 100 °C.

Das erfindungsgemäßen Verfahren wird im allgemeinen bei Normaldruck durchgeführt.

Zur Durchführung des erfindungsgemäßen Verfahrens setzt man je Mol 2-Alkylsulfonyl-pyrimidin-Derivat der Formel

(II) im allgemeinen zwischen 1 und 2 Mol, vorzugsweise zwischen 1,1 und 1,5 Mol Cyanamid oder die entsprechende Menge eines Metallsalzes hiervon ein. Die Reaktionskomponenten werden gewöhnlich bei Raumtemperatur oder unter leichtem Kühlen zusammen gegeben und bis zum Reaktionsende gerührt.

Zur Aufarbeitung wird das Reaktionsgemisch - gegebenenfalls nach Einengen - mit Wasser verrührt, mit einer Säure wie z. B. Essigsäure, Salzsäure oder Schwefelsäure, auf einen pH-Wert zwischen 1 und 7 gebracht und das hierbei kristallin anfallende Produkt der Formel (I) durch Absaugen isoliert.

Die nach dem erfindungsgemäßen Verfahren herzustellenden 2-Cyanamino-pyrimidin-Derivate der Formel (I) können als Zwischenprodukte für die Herstellung von bekannten Herbiziden und Pflanzenwachstumsregulatoren verwendet werden (vergl. DE-OS 3 334 455; EP-A-0 121 082).

## Herstellungsbeispiele

### Beispiel 1

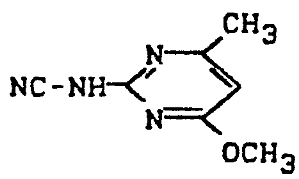

Eine Mischung aus 10,1 g (0,05 Mol) 4-Methoxy-6-methyl-2-methylsulfonyl-pyrimidin, 2,5 g (0,06 Mol) Cyanamid und 100 ml Dimethylformamid wird unter Rühren bei 20 °C mit 8,0 g (0,06 Mol) Kaliumcarbonat versetzt und das Reaktionsgemisch wird 6 Stunden bei 20 °C gerührt. Dann wird eingeengt, der Rückstand mit 150 ml Wasser verrührt und durch Zugabe von Essigsäure der pH-Wert auf 6,5 eingestellt. Das kristallin angefallene Produkt wird durch Absaugen isoliert.

Man erhält 6,4 g (78 % der Theorie) 2-Cyanamino-4-methoxy-6-methyl-pyrimidin vom Schmelzpunkt 258 °C (Zers.).

Analog können die in der nachstehenden Tabelle 1 aufgeführten Verbindungen der Formel (I) hergestellt werden:

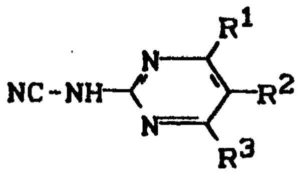

(I)

Tabelle 1

| Beisp.-Nr. | $R^1$ | $R^2$ | $R^3$ | Schmelzpunkt [° C] |
|---|---|---|---|---|
| 2 | $-OCH_3$ | H | $-OCH_3$ | 202 |
| 3 | H | H | $-CH_3$ | |
| 4 | $-CH_3$ | H | $-OCHF_2$ | 174 |
| 5 | -Cl | H | $-OCH_3$ | 202 (Zers.) |
| 6 | -Cl | H | $-CH_3$ | |
| 7 | -Cl | H | $-OC_2H_5$ | 169 |
| 8 | $-CH_3$ | H | $-OC_2H_5$ | 165 |
| 9 | H | H | $-C_2H_5$ | |
| 10 | $-OC_2H_5$ | H | $-OC_2H_5$ | 235 |

Tabelle 1 - Fortsetzung

| Beisp.-Nr. | $R^1$ | $R^2$ | $R^3$ | Schmelzpunkt [°C] |
|---|---|---|---|---|
| 11 | -Cl | -Cl | $-CH_3$ | |
| 12 | $-CH_3$ | -Cl | $-OCH_3$ | 225 |
| 13 | $-CH_3$ | -Cl | $-OC_2H_5$ | 212 |
| 14 | -Cl | -Cl | $-OCH_3$ | |
| 15 | H | H | $-OCH_3$ | amorph |
| 16 | -Cl | H | -Cl | |
| 17 | $-C_2H_5$ | H | $-OCH_3$ | 173 |
| 18 | $-OCH_3$ | -Cl | $-OCH_3$ | 218 |
| 19 | $-OC_2H_5$ | -Cl | $-OC_2H_5$ | 120 |
| 20 | $-CH_3$ | H | $-OC_3H_7$-i | 174 |

## Herstellung von Ausgangsstoffen der Formel (II)

**Beispiel (II-1)**

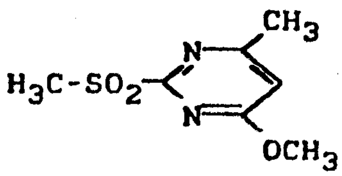

6,8 g (0,04 Mol) 4-Methoxy-6-methyl-2-methylthio-pyrimidin werden in ein Zweiphasensystem aus 50 ml Wasser und 100 ml Chloroform gegeben und in diese Mischung wird unter Rühren bei -5 °C bis +5 °C Chlor so lange eingeleitet, bis auch ohne weitere Chlor-Zufuhr das Reaktionsgemisch gelb gefärbt bleibt. Nach kurzem Nachrühren unter Durchleiten von Luft wird die organische Phase abgetrennt, mit Wasser gewaschen, getrocknet, filtriert und im Wasserstrahlvakuum eingeengt.

Man erhält 6,9 g (85 % der Theorie) 4-Methoxy-6-methyl-2-methylsulfonyl-pyrimidin als kristallinen Rückstand vom Schmelzpunkt 80 °C.

Analog können die in der nachstehenden Tabelle 2 aufgeführten Verbindungen der Formel (II) hergestellt werden:

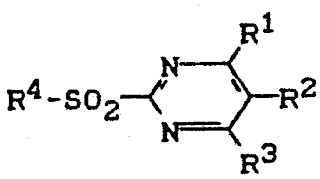

(II)

Tabelle 2

| Beisp.-Nr. | $R^1$ | $R^2$ | $R^3$ | $R^4$ | Schmelzpunkt [°C] |
|---|---|---|---|---|---|
| II-2 | $-OCH_3$ | H | $-OCH_3$ | $-CH_3$ | 130 |
| II-3 | H | H | $-CH_3$ | $-CH_3$ | |
| II-4 | H | H | $-OCH_3$ | $-CH_3$ | amorph |
| II-5 | $-CH_3$ | H | $-OCHF_2$ | $-CH_3$ | |
| II-6 | -Cl | H | $-OCH_3$ | $-CH_3$ | 86 |
| II-7 | -Cl | H | $-CH_3$ | $-CH_3$ | 72 |
| II-8 | $-CH_3$ | H | $-OC_2H_5$ | $-CH_3$ | amorph |
| II-9 | -Cl | H | $-OC_2H_5$ | $-CH_3$ | 109 |
| II-10 | $-OC_2H_5$ | H | $-OC_2H_5$ | $-CH_3$ | |
| II-11 | -Cl | -Cl | $-CH_3$ | $-C_2H_5$ | |

Tabelle 2 -Fortsetzung

| Beisp.-Nr. | $R^1$ | $R^2$ | $R^3$ | $R^4$ | Schmelzpunkt [°C] |
|---|---|---|---|---|---|
| II-12 | $-CH_3$ | -Cl | $-OCH_3$ | $-CH_3$ | 92 |
| II-13 | $-CH_3$ | -Cl | $-OC_2H_5$ | $-CH_3$ | 103 |
| II-14 | -Cl | H | -Cl | $-CH_3$ | 123 |
| II-15 | $-CH_3$ | -Cl | -Cl | $-CH_3$ | 97 |
| II-16 | $-C_2H_5$ | H | $-OCH_3$ | $-CH_3$ | amorph |
| II-17 | $-OCH_3$ | -Cl | $-OCH_3$ | $-CH_3$ | 191 |
| II-18 | $-OC_2H_5$ | -Cl | $-OC_2H_5$ | $-CH_3$ | 110 |

## Patentansprüche

1. Verfahren zur Herstellung von 2-Cyanamino-pyrimidin-Derivaten der allgemeinen Formel (I)

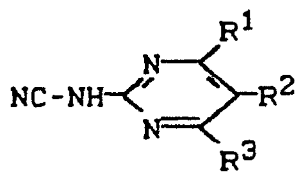

(I)

in welcher

$R^1$ für Wasserstoff, Halogen, gegebenenfalls substituiertes Alkyl oder gegebenenfalls substituiertes Alkoxy steht,

$R^2$ für Wasserstoff, Alkyl oder Halogen steht und

$R^3$ für Wasserstoff, Halogen, gegebenenfalls substituiertes Alkyl oder gegebenenfalls substituiertes Alkoxy steht,

dadurch gekennzeichnet, daß man 2-Alkylsulfonyl-pyrimidin-Derivate der allgemeinen Formel (II)

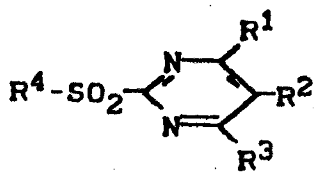 (II)

in welcher

$R^1$, $R^2$ und $R^3$ die oben angegebenen Bedeutungen haben und

$R^4$ für gegebenenfalls substituiertes Alkyl steht,

mit Cyanamid oder mit Metallsalzen von Cyanamid,

gegebenenfalls in Gegenwart eines Katalysators, gegebenenfalls in Gegenwart eines Säureakzeptors und gegebenenfalls in Gegenwart eines Verdünnungsmittels bei Temperaturen zwischen 0 und 150 °C umsetzt.

2) Verfahren nach Anspruch 1, dadurch gekennzeichnet, daß die Umsetzung bei Temperaturen zwischen 10 und 100°C durchgegührt wird.

3) Verfahren nach Anspruch 1, dadurch gekennzeichnet, als Verdünnungsmittel ein aprotisch polares Lösungsmittel eingesetzt wird.

4) Verfahren nach Anspruch 3, dadurch gekennzeichnet, daß als aprotisch polares Lösungsmittel Dimethylformamid, Dimethylacetamid, Acetonitril oder Propionitril eingesetzt wird.

5) Verfahren nach Anspruch 1, dadurch gekennzeichnet, daß auf 1 Mol 2-Alkylsulfonyl-pyrimidin-Derivat (II) zwischen 1 und 2 Mol, vorzugsweise zwischen 1,1 und 1,5 Mol, Cyanamid oder die entsprechende Menge eines Cyanamid-Metallsalzes eingesetzt werden.